# EUROPEAN PATENT SPECIFICATION

(11) **EP 2 800 518 B1**
(45) Date of publication and mention of the grant of the patent: **10.06.2020**
(21) Application number: 13733763.0
(22) Date of filing: 08.01.2013
(51) Int. Cl.: A61B 6/04

(54) **X-RAY COMPATIBLE PATIENT SUPPORT APPARATUS**
RÖNTGENSTRAHLKOMPATIBLE PATIENTENLIEGE
APPAREIL DE SUPPORT DE PATIENT COMPATIBLE AUX RAYONS X

(30) Priority: 08.01.2012 US 201261584295 P
(43) Date of publication of application: 12.11.2014
(73) Proprietor: Stryker Corporation, Kalamazoo, MI 49002 (US)
(72) Inventor: BROUGHAN, Matthew G., Kalamazoo, Michigan 49009 (US); CLIFFORD, Steven T., Grand Rapids, Michigan 49505 (US); SCHREIBER, Austin, Kalamazoo, Michigan 49006 (US); TESSMER, Brian J., Kalamazoo, Michigan 49004 (US); MCDANIEL, Richard L., Constantine, Michigan 49042 (US); BARTLEY, Gary L., Kalamazoo, Michigan 49004 (US); PATMORE, Kevin Mark, Plainwell, Michigan 49080 (US)
(74) Representative: FRKelly
(86) International application number: PCT/US2013/020625
(87) International publication number: WO 2013/104002

(56) References cited:
- KR-B1- 100 981 927
- US-A- 4 926 457
- US-A- 5 127 034
- US-A- 5 563 926
- US-A- 5 658 003
- US-A1- 2006 031 990
- US-B1- 6 341 398
- US-B1- 6 862 762

## Description

### BACKGROUND OF THE INVENTION

The present invention relates to patient support apparatuses, such as beds, stretchers, cots, gurneys, operating tables, and the like; and more particularly to patient support apparatuses that are configured to be compatible with diagnostic imaging systems, such as X-rays.

In a hospital setting, it is often desirable to take an X-ray, or other diagnostic image, of a patient without having to transfer him or her from the support apparatus that they are currently positioned on to another support apparatus dedicated to taking the diagnostic image. For example, in an emergency room, a patient is usually positioned on a stretcher or cot. If an X-ray is desired of the patient, it is generally disfavored to have to transfer the patient from the stretcher or cot to an X-ray machine support before being able to take the X-ray images. This is true for a number of reasons. First, the patient may have injuries that are exacerbated by the physical movement of the patient that may be necessary to transfer him or her to the X-ray machine support. Second, transferring the patient takes extra time and labor, and in an emergency room, such extra time and labor may not be available, or may be more advantageously used for other purposes. It is therefore desirable to have a patient support, such as a stretcher or cot, or the like, that both provides the desired support for the patient while also allowing diagnostic images to be taken of the patient while still being positioned on the patient support apparatus.

In the past, some patient support apparatuses that have allowed diagnostic images to be taken while the patient is positioned thereon have suffered from disadvantages. Such disadvantages may include difficulty in inserting the diagnostic imaging cartridge (such as an X-ray cartridge) underneath the patient; greater potentials for damaging the imaging cartridge; the creation of undesired visual artifacts in the resulting image due to the construction of the patient support apparatus; difficulty in positioning the diagnostic imaging cartridge in alignment with the other component of the diagnostic imaging machine; and/or other difficulties. Exemplary prior art patient support apparatuses are known from US-A1-4,926,457 or US-B1-6,341,398.

### SUMMARY OF THE INVENTION

The present invention provides an X-ray compatible patient support apparatus that alleviates one or more of the difficulties associated with prior art X-ray compatible patient support apparatuses. In some embodiments, the patient support apparatus improves the ease at which imaging cartridges are inserted underneath the patient. In other embodiments, the patient support apparatus reduces visual artifacts in the diagnostic image. In still other embodiments, the patient support apparatus reduces the likelihood of breaking the imaging cartridge. In still other embodiments, the patient support apparatus improves the ease of aligning the imaging cartridge with the section of the patient's body that is to be imaged. In still further embodiments, any one or more of these features may be combined with any one or more others of these features, and/or with other features.

According to one embodiment, a patient support apparatus is provided that includes a base, a frame supported on the base, a patient support deck, and a cartridge support surface. The patient support deck is supported on the frame and adapted to support a patient. The cartridge support surface is positioned underneath the support deck and is adapted to support an X-ray cartridge thereon. The cartridge support surface includes an interior region and a peripheral region, and the interior region is generally flat and horizontal, while at least a portion of the peripheral region is beveled with respect to the interior region.

According to another embodiment, a patient support apparatus is provided that includes a base, a frame supported on the base, a patient support deck, and a cartridge support surface. The patient support deck is adapted to support a patient, and the cartridge support surface is positioned underneath the support deck and adapted to support an X-ray cartridge thereon. The cartridge support surface includes a head end, a foot end, a first side, and a second side. The patient support deck and the cartridge support surface are vertically spaced apart from each other and open along at least the first side in regions adjacent both the head end and the foot end, whereby an X-ray cartridge may be inserted between the patient support deck and the cartridge support surface from the first side both at the head end region and the foot end region.

According to another embodiment, a patient support apparatus is provided that includes a base, a frame supported on the base, a cartridge support surface, and a patient support deck. The cartridge support surface is adapted to support an X-ray cartridge thereon, and the patient support deck is positioned above the cartridge support surface and is adapted to support a patient. The patient support deck includes a first section that is pivotable at a joint about a first pivot axis between a generally horizontal orientation and a raised orientation. The first pivot axis is generally parallel to a plane defined by the cartridge support surface, and the joint is constructed such that, during pivoting of the first section to the raised orientation, sufficient space is maintained at the joint between the patient support deck and the cartridge support surface for an X-ray cartridge to be positioned at the joint without being damaged by the pivoting of the first section.

According to yet another embodiment, a patient support apparatus is provided that includes a base, a frame supported on the base, a cartridge support surface, and a patient support deck. The cartridge support surface is adapted to support an X-ray cartridge thereon, and the patient support deck is positioned above the cartridge support surface and adapted to support a patient thereon. The patient support deck includes a first section and a second section adjacent to the first section. The first section of the patient support deck is pivotable at a joint about a first pivot axis between a generally horizontal orientation and a raised orientation. The first section and the second sections are configured such that each of the first and second sections includes an overlapping portion that vertically overlaps the overlapping portion of the other of the first and second sections at the joint while the first section is in the generally horizontal orientation.

According to still another embodiment, a patient support apparatus is provided that includes a base, a frame supported on the base, a cartridge support surface, a patient support deck, a handle, and a cable. The cartridge support surface is adapted to support an X-ray cartridge thereon, and the patient support deck is positioned above the cartridge support surface and adapted to support a patient. The patient support deck includes a first section that is pivotable at a joint about a first pivot axis between a generally horizontal orientation and a raised orientation. The first pivot axis is generally parallel to a plane defined by the cartridge support surface. The handle is coupled to the first section and is movable between a first position in which the first section may pivot about the first pivot axis and a second position in which the first section is prevented from pivoting about the first pivot axis. The handle is positioned adjacent a top end of the first section. The cable is coupled to the handle and a pivot control mechanism located underneath the cartridge support surface. The cable is routed along a side of the first section from the handle to the pivot control mechanism.

According to other aspects, the patient support apparatus may be a stretcher in which the base includes a plurality of wheels that may be selectively locked and unlocked to allow rolling movement of the stretcher. The frame of the stretcher may be supported on the base by a pair of elevation adjustment mechanisms that are adapted to allow both a height and an angular orientation of the frame with respect to the base to be adjusted. In other embodiments, the patient support apparatus may be a cot, a bed, or other type of support device for supporting a patient.

In still other aspects, the pivot control mechanism may be a gas spring and the cable may be a Bowden cable. Multiple handles may be positioned on the first section, with each handle positioned adjacent an upper corner of the first section. The first section may include a central region having a substantially constant thickness that is free of any components related to the handle, the cable, or the pivot control mechanism whereby the central region does not create visual artifacts in any X-rays taken through the central region of the first section.

### BRIEF DESCRIPTION OF THE DRAWINGS

FIG. 1 is a perspective view of a top portion of a patient support apparatus according to one embodiment showing a head section of a patient support deck pivoted to a raised orientation;
FIG. 2 is a perspective view of the top portion of FIG. 1 shown with both the head section and a lower section of the patient support deck pivoted to a raised orientation;
FIG. 3 is plan view of the top portion of FIG. 1;
FIG. 4 is a sectional view taken along the line IV-IV in FIG. 3;
FIG. 4A is an enlarged view of a portion of FIG. 4;
FIG. 5 is an enlarged, side, elevational view of the joint between the head section and the lower section of the patient support deck;
FIG. 6 is a perspective view of components of the head section, illustrating the configuration of a pair of handles, a pair of cables, and a pivot control mechanism;
FIG. 7 is a partially exploded, perspective view of the components of the head section taken from an angle similar to that of FIG. 6;
FIG. 8 is a bottom view of the components of FIG. 6;
FIG. 9 is a plan view of the components of FIG. 6;
FIG. 10 is a sectional view of the components of FIG. 9 taken along the line X-X in FIG. 9;
FIG. 11 is an enlarged view of the section of FIG. 9 labeled XI;
FIG. 12 is a partial, perspective view of the components of FIG. 6 shown attached to the frame of the patient support apparatus; and
FIG. 13 is a side, elevational diagram of an illustrative patient support apparatus that may incorporate one or more of the features of the present invention.

### DETAILED DESCRIPTION OF THE EMBODIMENTS

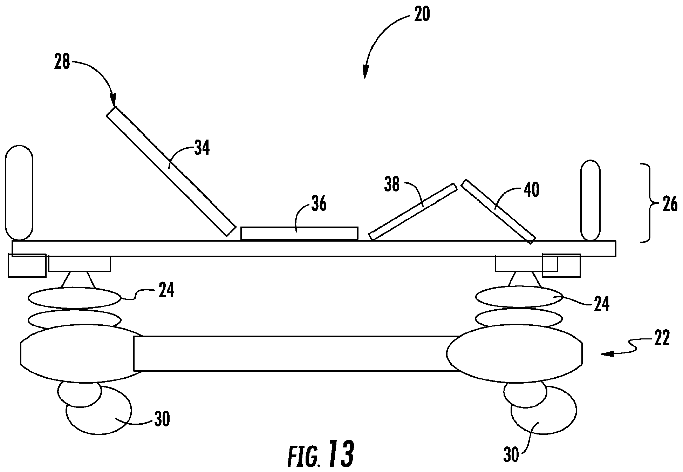

A diagram of a patient support apparatus 20 is illustrated in FIG. 13. Patient support apparatus 20 includes a base 22, a pair of elevation adjustment mechanisms 24, a frame or litter 26, and a patient support deck 28 that is supported on the frame. Base 22 includes a plurality of wheels 30 that are adapted to be selectively braked and unbraked to allow patient support apparatus 20 to be wheeled to different places. Elevation adjustment mechanisms 24 may be hydraulic cylinders, electric actuators, or any other type of actuating mechanism that is capable of adjusting the height of litter or frame 26 with respect to base 22. A control panel or other type of control (not shown) allows a patient and/or a caregiver to adjust both of the elevation adjustment mechanisms 24 is in unison so that the angle of litter 26 with respect to base 22 does not change. In one embodiment, the control panel or other type of control also allows the patient and/or caregiver to adjust the elevation adjustment mechanisms 24 individually so that the angle of the litter 26 with respect to the base 22 can be adjusted.

As shown in FIG. 13, patient support deck 28 includes a plurality of different sections, such as a head section 34, a seat section 36, a thigh section 38, and a foot section 40. In the illustrated embodiment, head section 34 is pivotable about a generally horizontal axis to enable the angle of head section 34 with respect to frame 26 to be adjusted. Other sections of patient support deck 28 may also be adjustable. Such adjustment is carried out with any suitable actuators, including electrical actuators or other types of actuators. Patient support deck 28 is adapted to provide a surface for supporting a patient on patient support apparatus 20. In most instances, a mattress, cushion, or other type of soft object (not shown) will be placed directly on top of patient support deck 28. A patient can then be supported on the mattress, or the like, on top of patient support deck 28.

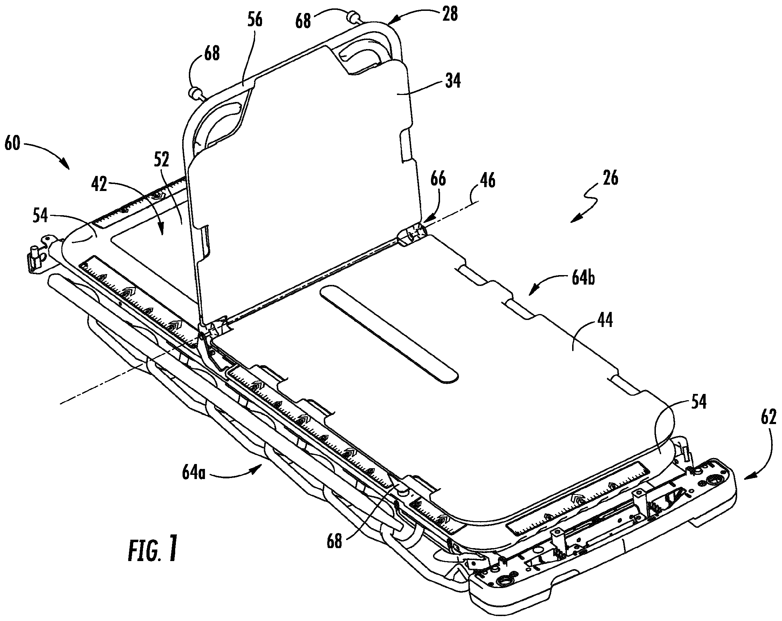

In many instances, as was noted above, it is desirable to be able to take an X-ray of a patient supported on patient support deck 28 without having to remove or transfer the patient from patient support apparatus 20. In order to do that, the frame or litter 26 of patient support apparatus 20 is specifically designed to enable such X-rays. One such litter 26, which can be incorporated into patient support apparatus 20, or any other type of patient support apparatus 20, is shown in FIGS. 1-4. Litter 26 can be used with the base 22 and elevation mechanisms 24 of FIG. 13, or it may be incorporated into other types of patient supports having different bases and/or different elevation mechanisms, as well as other patient support that omit an elevation mechanism altogether.

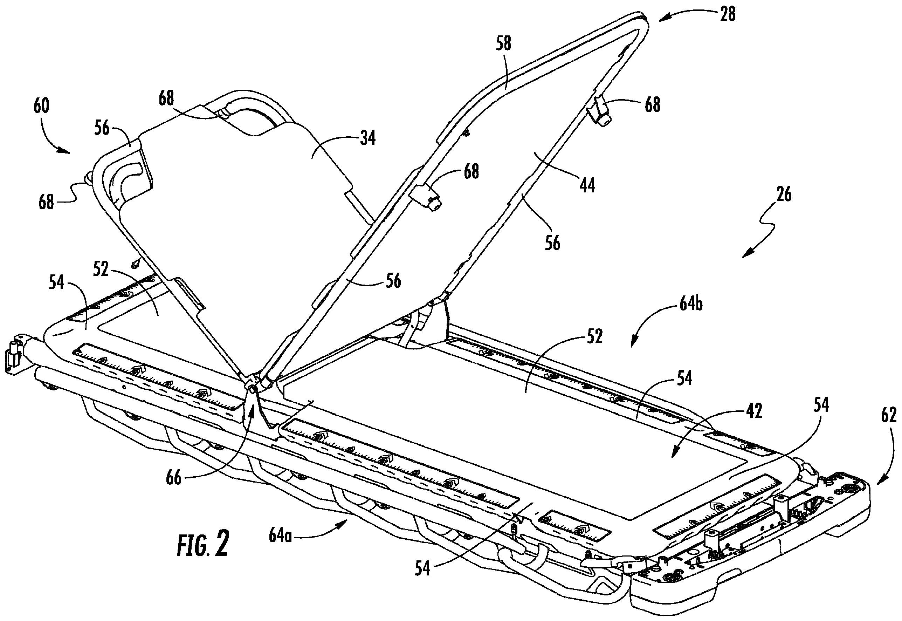

Frame 26 of FIGS. 1-4 includes a patient support deck 28 and a cartridge support surface 42 positioned underneath patient support deck 28. Patient support deck 28 in the embodiment of FIGS. 1-4 includes only two sections, unlike the patient support deck 28 of FIG. 13, which included four sections, although it will be understood that the frame 26 shown in FIGS. 1-4 could be modified to include more than two sections. As illustrated, patient support deck 28 includes an upper or head section 34 and a lower section 44. Both head section 34 and lower section 44 are pivotable about a generally horizontal pivot axis 46 positioned between the two sections. FIG. 1 shows head section 34 pivoted toward a raised orientation with lower section 44 in a generally horizontal orientation. FIG. 2 shows both head section 34 and lower section 44 pivoted toward a raised orientation. As noted above, sections 34 and 44 are adapted to support a mattress, cushion, or the like on which a patient may rest.

Cartridge support surface 42 is positioned underneath patient support deck 28 and is both generally planar and generally parallel to the plane defined by patient support deck 28 (when head section 34 and lower section 44 are lowered to their horizontal orientation). Cartridge support surface 42 is adapted to support a conventional diagnostic imaging cartridge thereon, such as, but not necessarily limited to, an X-ray cartridge. Conventional X-ray cartridges can be digital detectors that detect the X-rays passing through the patient (and support deck 28), or they can be non-digital X-ray cassettes. If digital, the cartridges may include a wire connected thereto that attaches the cartridge to a computer, or other electronic device, for processing the image data; or the digital cartridges may be wireless, in which case the cartridge wirelessly communicates the image data to a computer, display, or other electronic device.

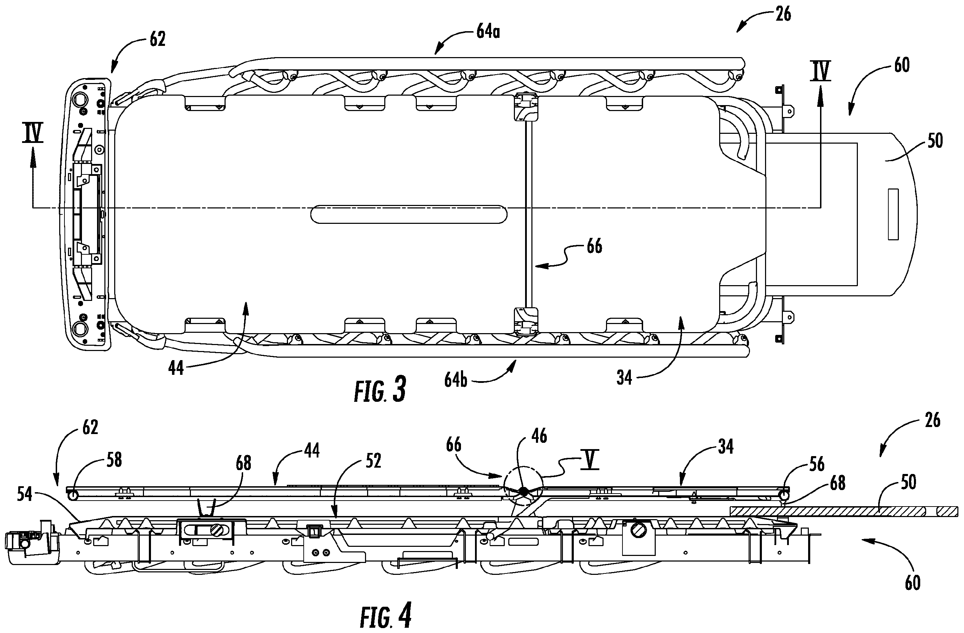

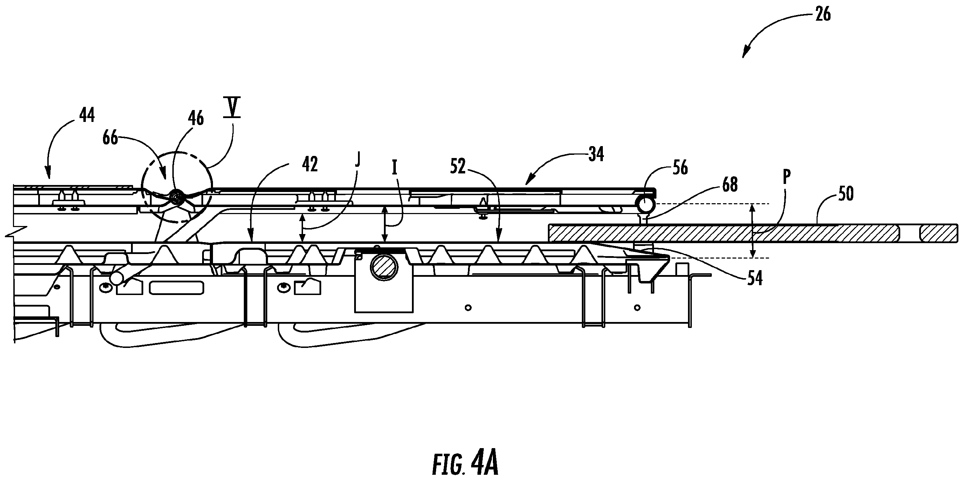

FIGS. 3 and 4 show one example of an X-ray cartridge 50 that is positionable on top of cartridge support surface 42 and underneath patient support deck 28. X-ray cartridge 50 is shown in FIGS. 3-4 as being positioned underneath head section 34 and on top of cartridge support surface 42. As noted, cartridge 50 is any conventional diagnostic cartridge. Such cartridges will have dimensions determined by the manufacturer of the cartridge, and such dimensions may vary from manufacturer to manufacturer. Usually, however, the cartridge manufacturers make cartridges that are approximately 35,56 cm by 43,18 cm (14 inches by 17 inches), with a height that varies from about 1,27 cm to about 2,54 cm (about a half of an inch to about an inch), depending upon the manufacturer of the cartridge and the type of cartridge that it is.

To take an X-ray of a patient supported on patient support deck 28, the cartridge 50 is inserted between patient support deck 28 and cartridge support surface 42. More particularly, cartridge 50 is positioned underneath the patient and on top of cartridge support surface 42. Cartridge 50 is positioned at a location on cartridge support surface 42 that lies underneath the portion of the patient that is to be X-rayed. An X-ray machine (not shown) is then moved into position above the patient and X-rays are emitted (generally vertically downward on the patient). The X-rays pass through the patient and patient support deck 28 until they reach cartridge 50. Cartridge 50 detects the X-rays and forms an image from the different amounts of X-ray absorption in the patient's body, as well as any different amounts of X-ray absorption in the components of patient support deck 28 that are positioned above cartridge 50 and that are aligned with the X-rays. Ideally, and as will be discussed more below, patient support deck 28 is constructed of a generally uniform thickness and uniform material so that its absorption of X-rays is uniform across the image and does not create any visual artifacts in the final image, particularly in areas that are likely to be of clinical interest to the caregivers.

As can be seen more clearly in FIGS. 1 and 2, cartridge support surface 42 includes an interior region 52 and a peripheral region 54. Interior region 52 is generally smooth, horizontal, and flat and is adapted to support the cartridge 50 thereon. Peripheral region 54, which surrounds interior region 52, is generally beveled with respect to interior region 52. That is, peripheral region 54 has an outer perimeter that is at a lower elevation than interior region 52. Moving from this outer perimeter inwardly toward interior region 52, the surface of peripheral region 54 slopes upwardly until it joins interior region 52. The upward beveling or angling of peripheral region 54 can be seen more clearly in FIG. 4A.

The beveled nature of peripheral region 54 helps assist in inserting a cartridge 50 between the bottom of patient support deck 28 and the top of cartridge support surface 42. This is because the beveled nature of peripheral region 54 helps create a greater vertical distance between the bottom of patient support deck 28 and the outer perimeter of peripheral region 54 than exists between the bottom of patient support deck 28 and the top of interior region 52. Or, stated alternatively and with specific reference to FIG. 4A, the beveled nature of peripheral region 54 makes distance P greater than distance I. Because distance P is greater, there is more space for a caregiver to insert cartridge 50 underneath a patient. The beveled nature of peripheral region 54 will cause the cartridge 50 to be moved upwardly toward the patient as the cartridge is fully inserted underneath the patient. This helps position the cartridge 50 closer to the patient, which is generally desirable when taking X-rays of the patient. While other dimensions may be used, in one embodiment, distance l may be about 5,08 cm (2 inches), while distance P may be about 7,62 cm, or even more than 7,62 cm (three inches, or even more than three inches).

It should be noted that, as shown in FIG. 4A, distance I represents the distance between the bottom of patient support deck 28 and the top of interior region 52 of cartridge support surface 42 that will be of interest when taking X-rays. Distance J, which is also shown in FIG. 4A, represents the distance between the top of interior region 52 and the bottom of an outer frame 56 of head section 34. Outer frame 56, however, is positioned around three sides of the perimeter of head section 34, and will normally not be an area where an X-ray image is desired.

As can be seen more clearly in FIG. 3, frame 26 includes a head end 60, a foot end 62, and first and second sides 64a and 64b. Except at a joint 66 and four support posts 68, there are no obstructions around the perimeter of cartridge support surface 42 and patient support deck 28 that prevent a caregiver from inserting a cartridge 50 between patient support deck 28 and cartridge support surface 42. A caregiver can thus easily slide a cartridge 50 to any area underneath the patient that is aligned with the area of the patient for which an X-ray, or other diagnostic image, is desired. This eliminates the difficulty associated with some prior art X-ray stretchers that only accept a cartridge at one specific location around the perimeter of the stretcher and, once the cartridge is inserted into the single entry point, require the cartridge to be slid from that entry point to the desired location underneath the patient. The embodiment of FIGS. 1-4 allows a cartridge to be inserted from the head end 60, the foot end 62 and from either side 64a and b, both near a foot region of sides 64a and b, and near a head region of sides 64a and b, as well as any locations intermediate the foot and head regions. Because joint 66 and support posts 68 are the only obstructions to inserting a cartridge underneath a patient, and these obstructions are few and spaced far apart, a caregiver has little difficulty in sliding the cartridge underneath the patient (and patient support deck 28) at the location that is aligned with, or close to being aligned with, the portion of the patient's body that is to be X-rayed.

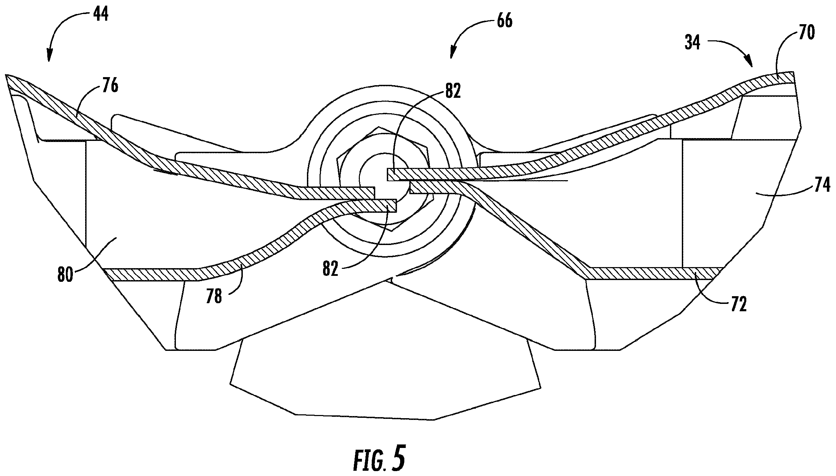

FIG. 5 shows a close up side, elevational view of joint 66. From FIG. 5, it can be seen that the main body of head section 34 is made up of an upper skin 70 and a lower skin 72 that is separated by a body 74. Similarly, the main body of lower section 44 is also made up of an upper skin 76 and a lower skin 78 that is separated by a body 80. In the case of upper section 34, the upper skin 70, lower skin 72, and body 74 are supported at their perimeter by outer frame 56. In the case of lower section 44, upper skin 76, lower skin 78, and body 80 are supported at their perimeter by outer frame 58 (FIG. 2). For both upper section 34 and lower section 44, the upper and lower skins may be made from a thermal formed plastic skin. In one embodiment, such thermal formed plastic skin is made from ABS (acrylonitrile butadiene styrene), although it may be made from some other suitable material. Both bodies 74 and 80 may be made of a suitable foam material, or a suitable urethane material. In one embodiment, bodies 74 and 80 are made from a polyiso foam or polyiso foam board. In other embodiments, other materials may be used.

Except at joint 66 and around the perimeters of head and lower sections 34 and 44, respectively, the thicknesses of head and lower sections 34 and 44 is substantially the same and constant. By having a non-varying thickness at all but the edges of sections 34 and 44, the amount of X-ray energy absorbed by sections 34 and 44 is generally uniform throughout their surface area. This helps reduce or eliminate visual artifacts that would otherwise show up in the resultant X-ray were different portions of sections 34 or 44 to be made of different thicknesses, or different materials, that absorbed different amounts of X-ray radiation than other portions of sections 34 and 44.

FIG. 5 shows the construction of sections 34 and 44 at joint 66 and illustrates how each section 34 and 44 includes an overlapping portion 82. Each overlapping portion 82 is made from a single layer of skin. In the case of head section 34, overlapping portion 82 is made of upper skin 70. In the case of lower section 44, overlapping portion 82 is made from lower skin 78. Overlapping portions 82 help ensure that, when head and lower sections 34 and 44 are both in the generally horizontal orientation, there is no region where an X-ray has to travel through four layers of skin, and there is also substantially no region where an X-ray does not have to travel through two layers of skin. More specifically, where overlapping portions 82 overlap, an X-ray will travel through upper skin 70 of upper section 34 and lower skin 78 of lower region 44. Thus, an X-ray will travel through two layers of skin at joint 66, just as an X-ray will travel through two layers of skin throughout substantially all of head section 34 and lower section 44 (except at the outer perimeters). Because an X-ray will travel through the same number of skin layers at joint 66 as it will when passing through the middle of upper or lower sections 34 or 44, the amount of X-ray absorption at joint 66 will be substantially the same as that at the middle of sections 34 and 44. Therefore, visual artifacts are substantially reduced at joint 66 when X-rays are taken that include joint 66. The construction of joint 66 and upper and lower sections 34 and 44 therefore help to reduce or eliminate visual artifacts in X-rays taken in this joint region.

It should be noted that, while FIG. 5 does show some small regions where an X-ray would only pass through a single layer of skin, such regions can be completely or substantially eliminated by extending one or both of overlapping regions 82 such that any such single-skin areas are completely or substantially eliminated.

Joint 66 is further constructed such that when either upper section 34 or lower section 44 is pivoted upwardly from the generally horizontal orientation, neither of the edges of sections 34 or 44 adjacent joint 66 get any closer to interior region 52 of cartridge support surface 42. This means that the clearance provided for a cartridge 50 positioned between patient support deck 28 and cartridge support surface 42 in the joint 66 region will not be reduced when either section 34 or 44 pivots. This, in turn, means that any cartridge positioned at joint 66 will not be pinched, cracked, or otherwise damaged, while one or both of sections 34 or 44 pivots upwardly from the horizontal orientation. This helps to prevent broken or damaged cartridges that may be partially or completely positioned near joint 66 while one or both of sections 34 and 44 are pivoted.

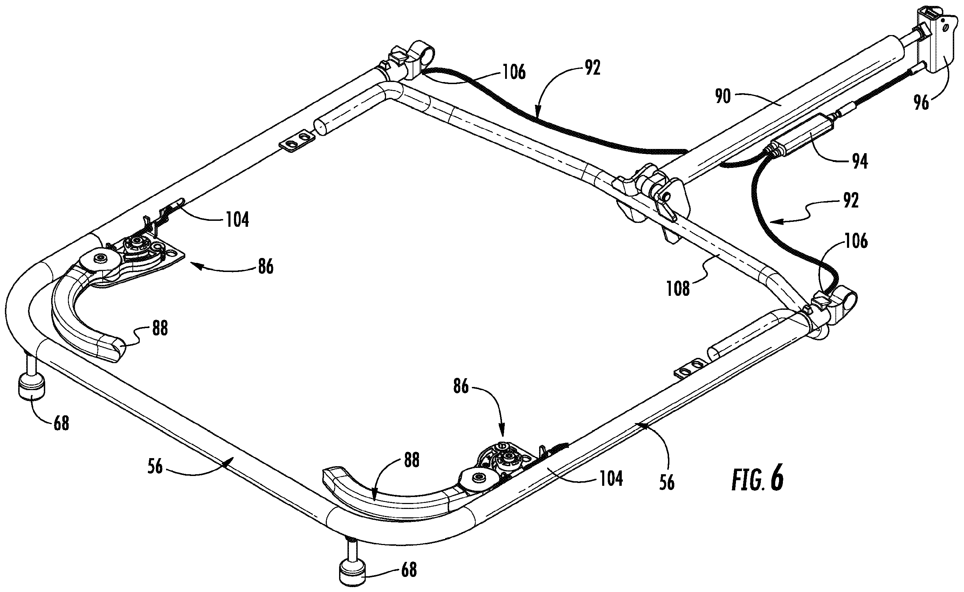

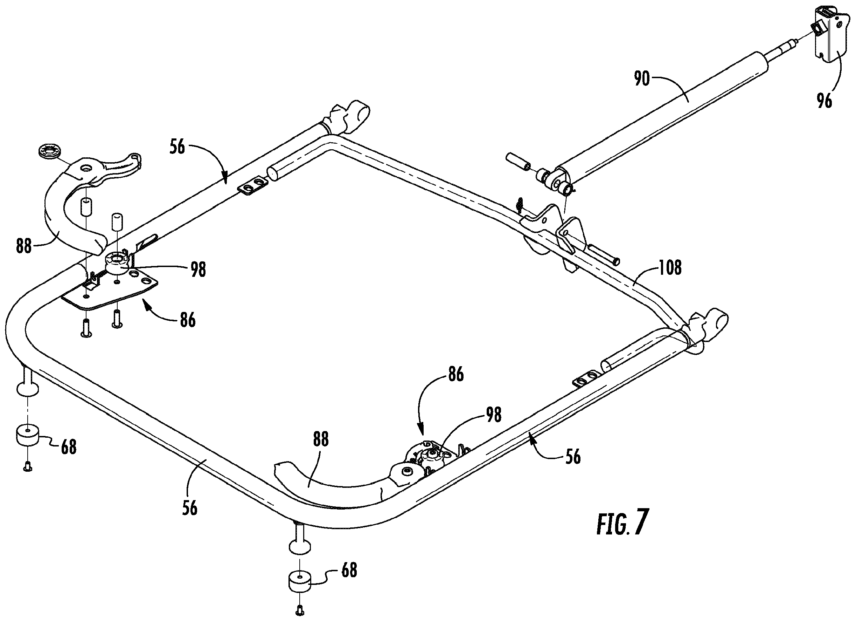

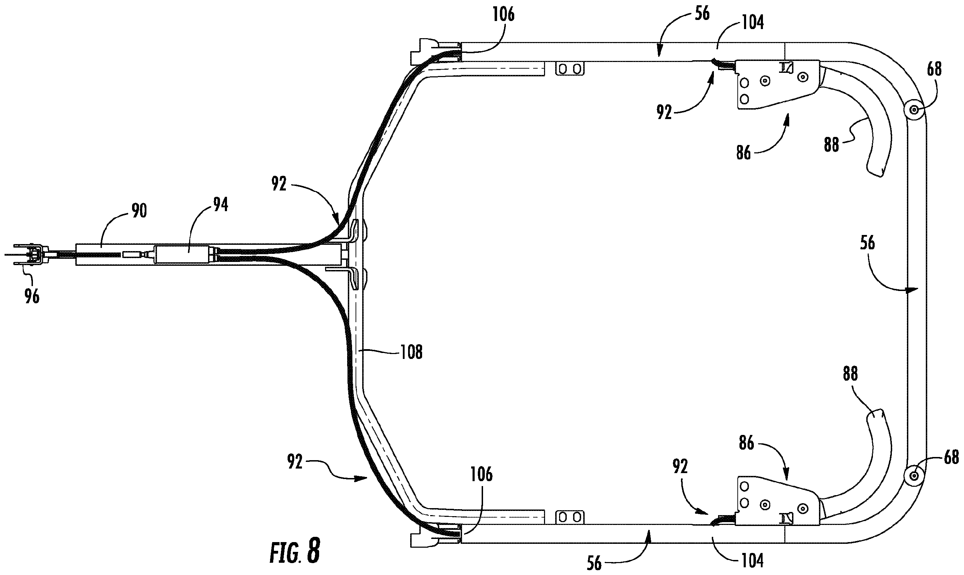

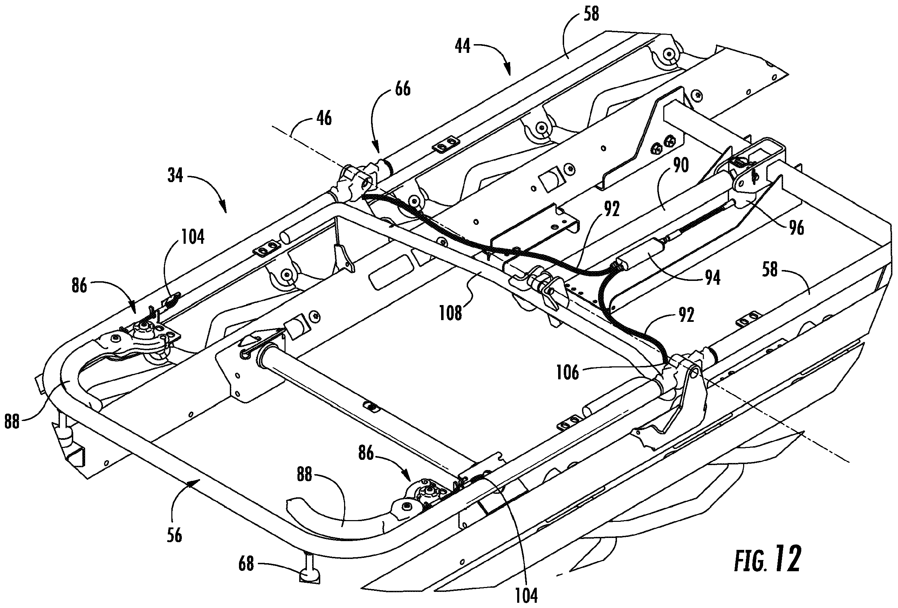

FIGS. 6-12 illustrate the construction of a pair of handles 88 that are attached to the upper end of upper section 34 adjacent each corner of upper section 34. Handles 88 are each adapted to be selectively squeezed by a caregiver. When one of handles 88 is squeezed, a handle control mechanism 86 coupled to the associated handle 88 transfers the motion of the handle 88 into a motion that is transmitted through one of a pair of cables 92. The cables are operatively coupled to a lock control structure 96 that releases a lock on a pivot control mechanism 90 positioned underneath cartridge support surface 42. The releasing of this lock enables upper section 34 to pivot about horizontal pivot axis 46. Or, stated alternatively, a caregiver must squeeze at least one of handles 88 if he or she wishes to pivot upper section 34 about axis 46. When both handles 88 are left unsqueezed, upper section 34 is locked in its current angular orientation with respect to cartridge support surface 42.

Pivot control mechanism 90 is, in one embodiment, a conventional gas spring that helps control the pivoting of head section 34 so that head section 34 is not free to simply fall downward when one of handles 88 is squeezed. In some embodiments, pivot control mechanism applies a force to head section 34 that helps resist downward pivoting of head section 34. In other embodiments, pivot control mechanism 90 simply dampens any movement and does not apply any positive forces itself. In still other embodiments, pivot control mechanism 90 is an electrical, or other type of, actuator that controls the movement of head section 34.

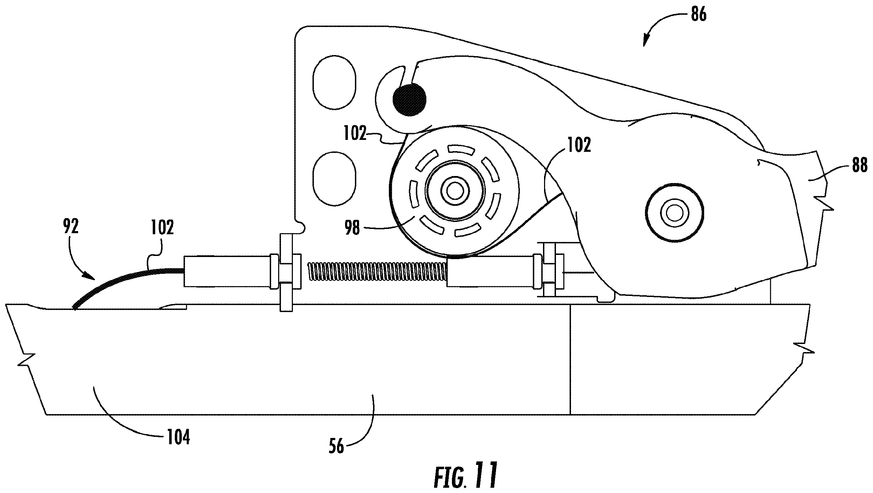

Each handle 88 controls the locking and unlocking of pivot control mechanism 90 by way of an associated cable 92 that runs between each handle 88 and a cable assembly 94. Cable assembly 94, in turn, is connected to lock control 96 of pivot control mechanism 90. Each cable 92 is a Bowden cable, or other type of mechanical cable, that includes an inner cable 100 surrounded by an outer sleeve 102 (FIG. 11). The inner cable is slidable longitudinally within the outer sleeve. Other types of mechanical cables, however, may alternatively be used.

As shown more clearly in FIG. 11, cable 92 is coupled to handle 88 by way of a pulley 98 around which an inner cable 100 of cable 92 is threaded. When handle 88 is pulled, inner cable 100 is also pulled, causing it to move around pulley 98 and to slide within outer sleeve 102. This movement of inner cable 100 is transmitted to cable assembly 94 which, in turn, transmits it to lock control 96. Cable assembly 94 is configured to transmit an unlocking force to lock control 96 whenever one or both of the inner cables 100 of cables 92 are pulled.

As can be seen more clearly in FIGS. 6-9, each cable 92 is threaded through a portion of outer frame 56. That is, frame 56 is a metal tubular structure having a hollow and generally circular or oval cross section. This hollow interior provides space for threading cable 92 therethrough from an upper location 104 adjacent to handles 88 to a lower location 106 near the terminal ends of outer frame 56. By threading cables 92 through these portions of outer frame 56, the cables 92 remain substantially out of the way of any portion of the patient's body that would likely be X-rayed, and also enable these portions of outer frame 56 to serve the dual purpose of both housing cables 92 and providing structural support to the body 74 and skins 70, 72 of head section 34.

The handle control mechanism 86, which is attached to each handle 88 and which converts the handle movement into a movement of the inner cable 100 of the Bowden cable, is relatively compact and confined to the corner regions of upper section 34. By keeping the handle control mechanisms 86 small, and by threading the cables 92 through the side portions of outer frame 56, the area on upper section 34 that could potentially create visual artifacts in the X-ray image is kept very small. That is, visual artifacts will only show up in the areas of handle control mechanisms 86 and the side edges of upper section 34. However, the handles 88 are positioned at the top edge of upper section 34 in an area where it is unlikely for any X-ray images to be taken. Further, even if an X-ray were taken at this high of a location on the patient's body, the patient's head would most likely be positioned between each handle control mechanism 86 so that neither handle control mechanism 86 would create visual artifacts in those areas of the X-ray corresponding to the patient's body. Further, any visual artifacts created along the side edges of upper section 34-due to either outer frame 56 and/or cables 92-would likely not be areas where the patient's body was positioned, so such artifacts would not affect the caregiver's diagnosis of the patient. In sum, upper section 34 is designed so that the vast majority of its interior space is free from structures that would tend to create any visual artifacts.

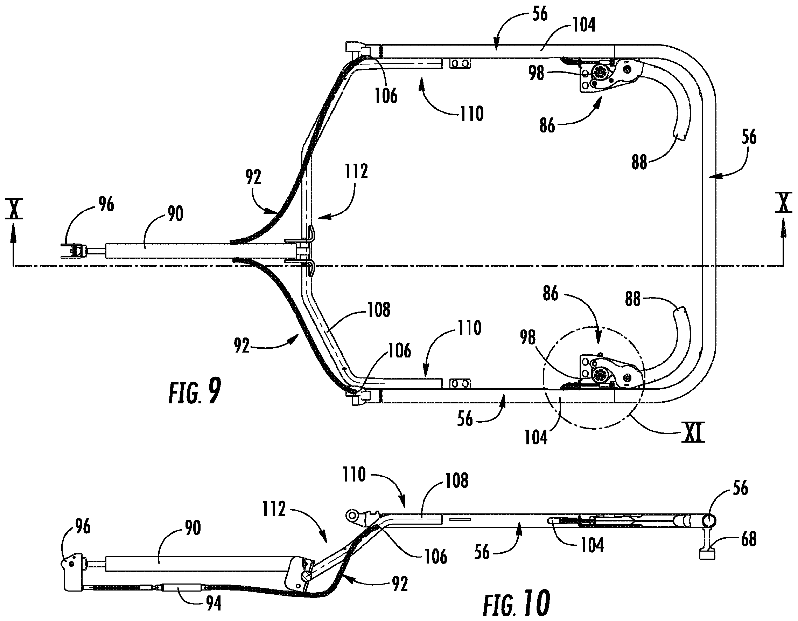

The connection of pivot control mechanism 90 to head section 34 is also constructed so that the potential for visual artifacts is substantially reduced, if not eliminated. This is accomplished in large part by positioning pivot control mechanism 90, cable assembly 94, lock control 96, and the majority of a cross bar 108 underneath cartridge support surface 42 so that these components are not positioned between the X-ray machine and the X-ray cartridge 50, thereby preventing them from interfering with the X-ray image. This is also accomplished by positioning those portions of cross bar 108 that do extend higher than cartridge support surface 42 along the extreme outer edges of the litter 26. As can be seen in FIGS. 9 and 10, cross bar 108 includes upper sections 110 that are secured to outer frame 56, such as by welding or other suitable fastening techniques, and a middle section 112 that is positioned underneath cartridge support surface 42 and which extends from one side of the patient support to the other. Upper sections 110 are positioned along the very sides of litter 26 where they will not interfere with an X ray cartridge 50, and middle section 112 is positioned underneath the X-ray cartridge support surface 42, where it also will not interfere with any X-ray images. Cross bar 108 therefore provides a structural support that links each of the two ends of outer frame 56 near joints 66, but does so in a manner that avoids interfering with the X-ray imagery. In combination, cross bar 108 and outer frame 56 form a complete and closed polygon whose interior portion is substantially covered by skins 70, 72 and body 74.

As can be seen in FIGS. 1-2, cartridge support surface 42 includes markings along peripheral region 54. These markings are provided to help a caregiver align the cartridge 50 with the patient's body and/or the X-ray machine. In some embodiments, the width of interior region 52 of cartridge support surface 42 (i.e. the distance from one side to the opposite side) is sized to be at least as long as a common length of conventional X-ray cartridges. For example, in one embodiment, interior region 52 has a width of approximately 17 inches, which corresponds to a common length of commercially available X-ray cartridges. Other dimensions may, of course, be used for interior region 52.

## Claims

1. A patient support apparatus (20) comprising:
a base (22);
a frame (26) supported on said base (22);
a cartridge support surface (42) supported on said frame (26) and adapted to support an X-ray cartridge thereon, said cartridge support surface (42) including a head end (60), a foot end (62), a first side (64a), and a second side (64b);
a patient support deck (28) supported on said frame (26) above said cartridge support surface (42), said patient support deck (28) adapted to support a patient, and said patient support deck (28) including a first section (34) and a second section (44), which are pivotable at a joint (66) about a first pivot axis (46) between a generally horizontal orientation and a raised orientation, said first pivot axis (46) being generally parallel to a plane defined by said cartridge support surface (42);
a handle (88) coupled to said first section (34) that is movable between a first position in which said first section (34) may pivot about said first pivot axis (46) and a second position in which said first section (34) is prevented from pivoting about said first pivot axis (46), said handle (88) being positioned adjacent the head end (60) of said first section (34); **characterized by**:
a cable (92) coupled to said handle (88) and a pivot control mechanism (90) located underneath said cartridge support surface (42), wherein said cable (92) is routed along a side (64) of said first section (34) from said handle (88) to said pivot control mechanism (90);
**characterized in that** said patient support deck (28) and said cartridge support surface (42) are vertically spaced apart from each other and open along both said first and second sides (64a, 64b) in regions adjacent both said head end (60) and said foot end (62), thereby allowing an X-ray cartridge (50) to be inserted between said patient support deck (28) and said cartridge support surface (42) from either said first side (64a) or said second side (64b) both at said head end (60) and said foot end (62); and
said cartridge support surface including an interior region and a peripheral region, said interior region being generally flat and horizontal, said peripheral region being beveled at the head end (60), at the foot end (62) and on the first and second sides (64a,64b) of said cartridge support surface, thereby allowing an X-ray cartridge (50), when the first section (34) and the second section (44) are lowered to their horizontal orientation, to be placed on top of said cartridge support surface (42) from any of the head end (60), the foot end (62), said first side (64a) and said second side (64b).

2. The apparatus (20) of claim 1 further including:
a second handle (88) coupled to said first section (34) that is movable between a first position in which said first section (34) may pivot about said first pivot axis (46) and a second position in which said first section (34) is prevented from pivoting about said first pivot axis (46), said second handle (88) being positioned adjacent the head end (60) of said first section (34); and
a second cable (92) coupled to said second handle (88) and said pivot control mechanism (90), wherein said second cable (92) is routed along the second side (64b) of said first section (34) from said second handle (88) to said pivot control mechanism (90).

3. The apparatus (20) of claim 2 wherein said pivot control mechanism (90) is a gas spring, and said cable (92) and said second cable (92) are Bowden cables (92).

4. The apparatus (20) of claim 3 wherein said handles (88) are positioned in opposite corners corner of said first section (34) and each includes a pulley (98) adapted to move an inner cable (100) of said Bowden cable (92) with respect to an outer sleeve (102) of said Bowden cable (92) when said respective handle (88) is moved.

5. The apparatus (20) of claim 1 wherein said first section (34) includes a central region having substantially constant thickness that is free of any components related to said handle (88), said cable (92), or said pivot control mechanism (90) whereby said central region does not create visual artifacts in any X-rays taken through said central region of said first section (34).

6. The apparatus (20) of claim 1 wherein said joint (66) is constructed such that, during pivoting of said first section (34) to the raised orientation, sufficient space is maintained at said joint (66) between said patient support deck (28) and said cartridge support surface (42) for an X-ray cartridge (50) to be positioned at said joint (66) without interfering with the pivoting of the first section (34).

7. The apparatus (20) of claim 1 wherein said first section and said second sections (34, 44) are configured such that each of said first and second sections (34, 44) includes an overlapping portion (82) that vertically overlaps the other of said first and second sections (34, 44) at said joint (66) while said first section (34) is in the generally horizontal orientation.

8. The apparatus (20) of claim 1 wherein said patient support apparatus (20) is a stretcher, said base (22) includes a plurality of wheels (30) that may be selectively locked and unlocked to allow rolling movement of said stretcher, and said frame (26) is supported on said base (22) by a pair of elevation adjustment mechanisms (24) that are adapted to allow both a height and an angular orientation of said frame (26) with respect to said base (22) to be adjusted.

9. The apparatus (20) of claim 7 wherein said overlapping portion (82) of said first and second sections (34, 44) has a thickness that is reduced compared to the rest of said first and second sections (34, 44).

10. The apparatus (20) of claim 9 wherein said first and second sections (34, 44) each include a top skin (70, 76) and a bottom skin (72, 78) in all areas except in said overlapping portion (82), said first and second sections (34, 44) each including only one of said top and bottom skins (70, 76, 72, 78) in said overlapping portion (82).

11. The apparatus (20) of claim 10 wherein said top and bottom skins (70, 76, 72, 78) are made from acrylonitrile butadiene styrene (ABS).

## Patentansprüche

1. Patientenlagerungsvorrichtung (20), umfassend:
ein Untergestell (22);
einen Rahmen (26), der auf dem Untergestell (22) gelagert ist;
eine Kassettenauflageoberfläche (42), die auf dem Rahmen (26) gelagert und dazu ausgelegt ist, eine Röntgenkassette darauf zu tragen, wobei die Kassettenauflageoberfläche (42) ein Kopfende (60), ein Fußende (62) und eine erste Seite (64a) und eine zweite Seite (64b) beinhaltet;
eine Patientenlagerungsfläche (28), die auf dem Rahmen (26) über der Kassettenauflageoberfläche (42) gelagert ist, wobei die Patientenlagerungsfläche (28) dazu ausgelegt ist, einen Patienten zu tragen, und die Patientenlagerungsfläche (28) einen ersten Abschnitt (34) und einen zweiten Abschnitt (44) beinhaltet, die an einer Verbindung (66) um eine erste Schwenkachse (46) zwischen einer allgemein horizontalen Ausrichtung und einer angehobenen Ausrichtung schwenkbar sind, wobei die erste Schwenkachse (46) im Allgemeinen parallel zu einer Ebene ist, die durch die Kassettenauflageoberfläche (42) definiert ist;
einen mit dem ersten Abschnitt (34) gekoppelten Griff (88), der zwischen einer ersten Position, in der der erste Abschnitt (34) um die erste Schwenkachse (46) schwenken kann, und einer zweiten Position, in der der erste Abschnitt (34) daran gehindert wird, um die erste Schwenkachse (46) zu schwenken, bewegbar ist, wobei der Griff (88) neben dem Kopfende (60) des ersten Abschnitts (34) positioniert ist; **gekennzeichnet durch**:
ein Kabel (92), das mit dem Griff (88) gekoppelt ist, und einen Schwenksteuermechanismus (90), der sich unter der Kassettenauflageoberfläche (42) befindet, wobei das Kabel (92) entlang einer Seite (64) des ersten Abschnitts (34) von dem Griff (88) zu dem Schwenksteuermechanismus (90) geführt ist;
**dadurch gekennzeichnet, dass** die Patientenlagerungsfläche (28) und die Kassettenauflageoberfläche (42) vertikal voneinander beabstandet sind und entlang sowohl der ersten als auch der zweiten Seite (64a, 64b) in Bereichen sowohl neben dem Kopfende (60) als auch dem Fußende (62) offen sind, wodurch ermöglicht wird, dass eine Röntgenkassette (50) zwischen der Patientenlagerungsfläche (28) und der Kassettenauflageoberfläche (42) entweder von der ersten Seite (64a) oder der zweiten Seite (64b) sowohl an dem Kopfende (60) als auch an dem Fußende (62) eingeführt werden kann; und
wobei die Kassettenauflageoberfläche einen Innenbereich und einen Umfangsbereich beinhaltet, wobei der Innenbereich im Allgemeinen flach und horizontal ist, der Umfangsbereich an dem Kopfende (60), an dem Fußende (62) und an der ersten und der zweiten Seite (64a, 64b) der Kassettenauflageoberfläche abgeschrägt ist, wodurch ermöglicht wird, dass, wenn der erste Abschnitt (34) und der zweite Abschnitt (44) in ihre horizontale Ausrichtung abgesenkt werden, eine Röntgenkassette (50) von einem beliebigen von dem Kopfende (60), dem Fußende (62), der ersten Seite (64a) und der zweiten Seite (64b) auf die Kassettenauflageoberfläche (42) gelegt werden kann.

2. Vorrichtung (20) nach Anspruch 1, ferner beinhaltend:
einen mit dem ersten Abschnitt (34) gekoppelten zweiten Griff (88), der zwischen einer ersten Position, in der der erste Abschnitt (34) um die erste Schwenkachse (46) schwenken kann, und einer zweiten Position, in der der erste Abschnitt (34) daran gehindert wird, um die erste Schwenkachse (46) zu schwenken, bewegbar ist, wobei der zweite Griff (88) neben dem Kopfende (60) des ersten Abschnitts (34) positioniert ist; und
ein zweites Kabel (92), das mit dem zweiten Griff (88) und dem Schwenksteuermechanismus (90) gekoppelt ist, wobei das zweite Kabel (92) entlang der zweiten Seite (64b) des ersten Abschnitts (34) von dem zweiten Griff (88) zu dem Schwenksteuermechanismus (90) geführt ist.

3. Vorrichtung (20) nach Anspruch 2, wobei der Schwenksteuermechanismus (90) eine Gasfeder ist, und das Kabel (92) und das zweite Kabel (92) Bowden-Kabel (92) sind.

4. Vorrichtung (20) nach Anspruch 3, wobei die Griffe (88) in gegenüberliegenden Ecken des ersten Abschnitts (34) positioniert sind und jeweils eine Riemenscheibe (98) beinhalten, die dazu ausgelegt ist, ein inneres Kabel (100) von dem Bowden-Kabel (92) in Bezug auf eine Außenhülse (102) des Bowden-Kabels (92) zu bewegen, wenn der jeweilige Griff (88) bewegt wird.

5. Vorrichtung (20) nach Anspruch 1, wobei der erste Abschnitt (34) einen zentralen Bereich beinhaltet, der eine im Wesentlichen konstante Dicke aufweist, der frei von Komponenten ist, die zu dem Griff (88), dem Kabel (92) oder dem Schwenksteuermechanismus (90) gehören, wodurch der zentrale Bereich keine visuellen Artefakte in Röntgenaufnahmen erzeugt, die durch den zentralen Bereich des ersten Abschnitts (34) aufgenommen wurden.

6. Vorrichtung (20) nach Anspruch 1, wobei die Verbindung (66) derart konstruiert ist, dass beim Schwenken des ersten Abschnitts (34) in die angehobene Ausrichtung ausreichend Raum an der Verbindung (66) zwischen der Patientenlagerungsfläche (28) und der Kassettenauflageoberfläche (42) aufrecht erhalten wird, dass eine Röntgenkassette (50) an der Verbindungstelle (66) positioniert werden kann, ohne das Schwenken des ersten Abschnitts (34) zu stören.

7. Vorrichtung (20) nach Anspruch 1, wobei der erste Abschnitt und der zweite Abschnitt (34, 44) derart konfiguriert sind, dass jeder von dem ersten und dem zweiten Abschnitt (34, 44) einen Überlappungsabschnitt (82) beinhaltet, der den anderen von dem ersten und dem zweiten Abschnitt (34, 44) an der Verbindung (66) vertikal überlappt, während sich der erste Abschnitt (34) in der allgemein horizontalen Ausrichtung befindet.

8. Vorrichtung (20) nach Anspruch 1, wobei die Patientenlagerungsvorrichtung (20) eine Tragbahre ist, wobei das Untergestell (22) eine Vielzahl von Rädern (30) beinhaltet, die selektiv gesperrt und entriegelt werden können, um eine Rollbewegung der Tragbahre zu ermöglichen, und der Rahmen (26) auf dem Untergestell (22) durch ein Paar von Höheneinstellmechanismen (24) getragen wird, die dazu ausgelegt sind, zu ermöglichen, dass sowohl eine Höhe als auch eine Winkelausrichtung des Rahmens (26) in Bezug auf das Untergestell (22) eingestellt werden kann.

9. Vorrichtung (20) nach Anspruch 7, wobei der Überlappungsabschnitt (82) des ersten und des zweiten Abschnitts (34, 44) eine im Vergleich zu dem Rest von dem ersten und dem zweiten Abschnitt (34, 44) geringere Dicke aufweist.

10. Vorrichtung (20) nach Anspruch 9, wobei der erste und der zweite Abschnitt (34, 44) jeweils eine obere Außenhaut (70, 76) und eine untere Außenhaut (72, 78) in allen Bereichen mit Ausnahme des Überlappungsabschnitts (82) beinhalten, wobei der erste und der zweite Abschnitt (34, 44) jeweils nur eine von der oberen und der unteren Außenhaut (70, 76, 72, 78) in dem Überlappungsabschnitt (82) beinhalten.

11. Vorrichtung (20) nach Anspruch 10, wobei die obere und die untere Außenhaut (70, 76, 72, 78) aus AcrylnitrilButadien-Styrol (ABS) hergestellt sind.

## Revendications

1. Appareil de support de patient (20) comprenant :
une base (22) ;
un cadre (26) supporté sur ladite base (22) ;
une surface de support de cartouche (42) supportée sur ledit cadre (26) et conçue pour supporter une cartouche de rayons X sur celle-ci, ladite surface de support de cartouche (42) comportant une extrémité de tête (60), une extrémité de pied (62), un premier côté (64a) et un second côté (64b) ;
une plateforme de support de patient (28) supportée sur ledit cadre (26) au-dessus de ladite surface de support de cartouche (42), ladite plateforme de support de patient (28) étant conçue pour supporter un patient, et ladite plateforme de support de patient (28) comportant une première section (34) et une seconde section (44), qui peuvent pivoter au niveau d'une articulation (66) autour d'un premier axe de pivotement (46) entre une orientation généralement horizontale et une orientation surélevée, ledit premier axe de pivotement (46) étant généralement parallèle à un plan défini par ladite surface de support de cartouche (42) ;
une poignée (88) couplée à ladite première section (34) qui est mobile entre une première position dans laquelle ladite première section (34) peut pivoter autour dudit premier axe de pivotement (46) et une seconde position dans laquelle ladite première section (34) ne peut pas pivoter autour dudit premier axe de pivotement (46), ladite poignée (88) étant positionnée de manière adjacente à l'extrémité de tête (60) de ladite première section (34) ; **caractérisé par** :
un câble (92) couplé à ladite poignée (88) et un mécanisme de commande de pivotement (90) situé sous ladite surface de support de cartouche (42), dans lequel ledit câble (92) est acheminé le long d'un côté (64) de ladite première section (34) à partir de ladite poignée (88) jusqu'audit mécanisme de commande de pivotement (90) ;
**caractérisé en ce que** ladite plateforme de support de patient (28) et ladite surface de support de cartouche (42) sont espacées verticalement l'une de l'autre et s'ouvrent à la fois le long desdits premier et second côtés (64a, 64b) dans des régions adjacentes à la fois à ladite extrémité de tête (60) et à ladite extrémité de pied (62), permettant ainsi à une cartouche de rayons X (50) d'être insérée entre ladite plateforme de support de patient (28) et ladite surface de support de cartouche (42) à partir dudit premier côté (64a) ou dudit second côté (64b) à la fois au niveau de ladite extrémité de tête (60) et de ladite extrémité de pied (62) ; et
ladite surface de support de cartouche comportant une région intérieure et une région périphérique, ladite région intérieure étant généralement plate et horizontale, ladite région périphérique étant biseautée au niveau de l'extrémité de tête (60), de l'extrémité de pied (62) et sur les premier et second côtés (64a, 64b) de ladite surface de support de cartouche, permettant ainsi à une cartouche de rayons X (50), lorsque la première section (34) et la seconde section (44) sont abaissées dans leur orientation horizontale, d'être placée au-dessus de ladite surface de support de cartouche (42) à partir de l'un quelconque parmi l'extrémité de tête (60), l'extrémité de pied (62), ledit premier côté (64a) et ledit second côté (64b).

2. Appareil (20) selon la revendication 1, comportant en outre :
une seconde poignée (88) couplée à ladite première section (34) qui est mobile entre une première position dans laquelle ladite première section (34) peut pivoter autour dudit premier axe de pivotement (46) et une seconde position dans laquelle ladite première section (34) ne peut pas pivoter autour dudit premier axe de pivotement (46), ladite seconde poignée (88) étant positionnée de manière adjacente à l'extrémité de tête (60) de ladite première section (34) ; et
un second câble (92) couplé à ladite seconde poignée (88) et audit mécanisme de commande de pivotement (90), dans lequel ledit second câble (92) est acheminé le long du second côté (64b) de ladite première section (34) à partir de ladite seconde poignée (88) jusqu'audit mécanisme de commande de pivotement (90).

3. Appareil (20) selon la revendication 2, dans lequel ledit mécanisme de commande de pivotement (90) est un ressort à gaz, et ledit câble (92) et ledit second câble (92) sont des câbles Bowden (92).

4. Appareil (20) selon la revendication 3, dans lequel lesdites poignées (88) sont positionnées dans le coin des coins opposés de ladite première section (34) et comportent chacune une poulie (98) conçue pour déplacer un câble intérieur (100) dudit câble Bowden (92) par rapport à un manchon extérieur (102) dudit câble Bowden (92) lorsque ladite poignée respective (88) est déplacée.

5. Appareil (20) selon la revendication 1, dans lequel ladite première section (34) comporte une région centrale ayant une épaisseur sensiblement constante qui est exempte de tout composant lié à ladite poignée (88), audit câble (92) ou audit mécanisme de commande de pivotement (90), ladite région centrale ne créant pas d'artefacts visuels dans les rayons X pris à travers ladite région centrale de ladite première section (34).

6. Appareil (20) selon la revendication 1, dans lequel ladite articulation (66) est construite de sorte que, lors du pivotement de ladite première section (34) vers l'orientation surélevée, un espace suffisant est maintenu au niveau de ladite articulation (66) entre ladite plateforme de support de patient (28) et ladite surface de support de cartouche (42) pour une cartouche de rayons X (50) à positionner au niveau de ladite articulation (66) sans interférer avec le pivotement de la première section (34).

7. Appareil (20) selon la revendication 1, dans lequel ladite première section et ladite seconde section (34, 44) sont configurées de sorte que chacune desdites première et seconde sections (34, 44) comporte une partie de chevauchement (82) qui chevauche verticalement l'autre desdites première et seconde sections (34, 44) au niveau de ladite articulation (66) tandis que ladite première section (34) est dans l'orientation généralement horizontale.

8. Appareil (20) selon la revendication 1, dans lequel ledit appareil de support de patient (20) est une civière, ladite base (22) comporte une pluralité de roues (30) qui peuvent être sélectivement verrouillées et déverrouillées pour permettre un mouvement de roulement de ladite civière, et ledit cadre (26) est supporté sur ladite base (22) par une paire de mécanismes de réglage d'élévation (24) qui sont conçus pour permettre à la fois le réglage d'une hauteur et d'une orientation angulaire dudit cadre (26) par rapport à ladite base (22).

9. Appareil (20) selon la revendication 7, dans lequel ladite partie de chevauchement (82) desdites première et seconde sections (34, 44) a une épaisseur qui est réduite par rapport au reste desdites première et seconde sections (34, 44) .

10. Appareil (20) selon la revendication 9, dans lequel lesdites première et seconde sections (34, 44) comportent chacune une peau supérieure (70, 76) et une peau inférieure (72, 78) dans toutes les zones sauf dans ladite partie de chevauchement (82), lesdites première et seconde sections (34, 44) comportant chacune uniquement l'une desdites peaux supérieure et inférieure (70, 76, 72, 78) dans ladite partie de chevauchement (82).

11. Appareil (20) selon la revendication 10, dans lequel lesdites peaux supérieure et inférieure (70, 76, 72, 78) sont fabriquées à partir d'acrylonitrile butadiène styrène (ABS).
